# EUROPÄISCHE PATENTANMELDUNG

(11) **EP 1 215 447 A2**
(43) Veröffentlichungstag der Anmeldung: **19.06.2002**
(21) Anmeldenummer: 01109575.9
(22) Anmeldetag: 18.04.2001
(51) Int. Cl.: F24F 3/12

(54) **Verfahren und Vorrichtung zur Duftsteuerung**

(30) Priorität: 15.12.2000 DE 10062630
(71) Anmelder: Bartels Mikrotechnik GmbH, 44227 Dortmund (DE)
(72) Erfinder: Bartels, Frank, Dr., 44227 Dortmund (DE); Prahl, Helmut, 22143 Hamburg (DE)
(74) Vertreter: Pätzold, Herbert, Dr.

(57) **Zusammenfassung**

Verfahren zum Erzeugen und/oder zur Neutralisierung von Düften mittels in einem flüssigen Medium enthaltenen Duftstoffen mit im wesentlichen drei Verfahrensschritten.

Kollision der Duftstoffe unter hohem Druck aus einer geeigneten Düse in einen nachgeschalteten Reaktionsraum, so dass ein Aerosol erzeugt wird.

Bereitstellung von Mitteln zum Transport des Aerosols aus dem Reaktionsraum und Transport des Aerosols aus dem Reaktionsraum.

Nach einer vorteilhaften Ausführung des Verfahrens wird in dem nachgeschalteten Reaktionsraum Energie bereitgestellt, so dass eine weitere Diffusion des Aerosols nach dem Düsenaustritt erfolgt.

Außerdem: Vorrichtung zur Durchführung des Verfahrens, die insbesondere ein mikroelektromechanisches Bauteil zur Ausbringung der Duftstoffe in den Reaktionsraum aufweist, und außerdem geeignete Mittel zum Transport des Aerosols aus dem Reaktionsraum aufweist, und nach einer vorteilhaften Ausführung außerdem einen geeigneten Energiegenerator zur Bereitstellung der Energie für die Nachbehandlung der Duftstoffe in dem Reaktionsraum umfaßt.

## Beschreibung

Die vorliegenden Erfindung betrifft ein Verfahren und eine Vorrichtung zur Duftsteuerung.

Aktive Beduftung spielt nicht nur in der Informations- und Kommunikationstechnologie eine zunehmende Rolle. Düfte wirken nämlich direkt auf die Empfindungen des Menschen und werden nicht wie andere Sinneswahrnehmungen durch unseren Verstand gefiltert. Außerdem können Düfte menschliche Emotionen und Empfindungen beträchtlich beeinflussen. Eine aktive Kontrolle des Duftes der menschlichen Umgebung ist daher immer dann von Interesse, wenn durch die Entfernung unangenehmer und/oder durch Hinzufügen von angenehmen Düften eine Verbesserung bzw. Veränderung des Befindens erzeugt werden soll. Dies gilt für alle Räume, in denen wir uns längere Zeit aufhalten, wie z. B. Wohn- und Schlafräume, Arbeitsplatz, Auto usw. Außerdem werden zunehmend in der Werbetechnik Düfte eingesetzt, um Produkteigenschaften zu präsentieren, wobei ein für das Produkt werbender und gleichzeitig das Produkt charakterisierender Duft zusammen mit dem Produkt angeboten wird. Auch im Multimediabereich und in Kinos werden Düfte im zunehmenden Maß in Kombination mit Ton und Bild eingesetzt, wobei durch den Dufteinsatz eine Erhöhung der Aufmerksamkeit des Konsumenten und der Intensität seiner Empfindungen bei der Aufnahme von Informationen und Unterhaltung erzielt wird.

Herkömmliche Techniken zum Ausbringen von vorbestimmten Duftstoffen oder zur gezielten Vernichtung von Düften arbeiten beispielsweise auf dem Prinzip von Tintenstrahldruckern, bei dem eine vorbestimmte Menge einer Flüssigkeit in kleinen Tropfen in die Luft gespritzt werden. Die dabei zum Austritt der Flüssigkeit verwendeten Düsen haben jedoch derart große Öffnungen zum Austritt der Flüssigkeit, so dass bei Ruhebetrieb der Düse, die an der Düsenöffnung befindliche Flüssigkeit leicht eintrocknet. Dieses Problem wurde bei Tintenstrahldruckern durch die Entwicklung von Tinten mit sehr geringen Verdampfungsraten gelöst, was bei der Ausbringung von Duftstoffen jedoch prinzipiell nicht möglich ist, da die Duftstoffe funktionsbedingt in der Luft verdampfen sollen. Außerdem wird durch teilweises Verdampfen der Flüssigkeit im Ruhebetrieb die Qualität der Duftausbringung bei erneutem Arbeitseinsatz der Düse herabgesetzt, da sich in Flüssigkeiten gelöste Düfte an der Grenzfläche Luft/Flüssigkeit leicht verändern. Außerdem werden mit den herkömmlichen Verfahren Tropfen im Bereich oberhalb von etwa 10 µm Teilchendurchmesser erzeugt, was zu einer nachteiligen Beeinflussung der Tröpfchen untereinander führt und auch zu einer nachteiligen Benetzung von Oberflächen im Absenkbereich der Tropfen. Derartige Systeme zum Ausbringen von Düften sind daher insbesondere wenn Dufteindrücke schnell geändert werden sollen, nicht kontrolliert einsetzbar.

Aus dem Stand der Technik sind außerdem thermische Verdampfer bekannt, die aus einem Flüssigkeitsreservoir durch Temperaturerhöhung eine vorbestimmte Duftmenge entlassen, wobei diese Duftstoffmenge vollständig verdampft. Die thermischen Verdampfer erzeugen zwar Duftstoffe mit kleinsten Teilchen, die nicht so leicht wie die zuvor beschriebenen Verfahren eine nachteilige Benetzung von Oberflächen der näheren Umgebung mit den damit verbundenen zuvor beschriebenen Nachteilen verursachen. Die auf Temperaturerhöhung beruhenden Systeme erfordern jedoch besonders temperaturfeste Duftstoffe, was den Bereich der Duftstoffverwendung und damit den Einsatz dieser Systeme beträchtlich einschränkt.

Zusammenfassend wird hier festgestellt, dass vorhandene Verfahren und Geräte zur Duftwiedergabe in ihrer Bandbreite und Qualität der Duftwiedergabe eng limitiert sind. Außerdem lassen die herkömmlichen Verfahren, Geräte und Systeme eine Mischung von einzelnen Duftölen nicht zu, sind ungenügend regelbar und erzeugen einen für viele Anwendungen nur unzureichende Reichweite des Luft/Duftstroms. Daher ist der mittels dem Stand der Technik erzeugbare Dufteindruck häufig durch Wärmeeinwirkung, Dufttransfers und chemische Reaktionen verfälscht.

Aus dem Stand der Technik aus DE-42 36 037 A1 ist außerdem ein Düsenkörper zur Verteilung von Arzneimitteln bekannt, die zur Aufnahme in der Lunge bestimmt sind. Dieser Düsenkörper wurde von einem der Anmelder der vorliegenden Erfindung in der unveröffentlichten deutschen Patentanmeldung Nr. 100 62 630.0 für die Verwendung in einem Verfahren zur Erzeugung von Düften weitergebildet und außerdem wurde in dieser Anmeldung eine Anordnung zur Erzeugung von Düften vorgeschlagen, bei der ebenfalls die aus der DE-42 36 037 A1 bekannte Düse und die gemäß der obigen deutschen Patentanmeldung 100 62 630.0 weitergebildete Düse eingesetzt wird.

Die Düse gemäß DE-42 36 037 A1 erzielt zwar Tropfen in einer Größenordnung, die grundsätzlich für die Verwendung der Düse im Bereich von Dufttechniken geeignet erscheint. Die Tropfen sind jedoch nachteilhaft als stehende Aerosolwolke vorhanden, und außerdem ist mit Verwendung allein der Düse zur Erzeugung von Aerosolen nicht immer sichergestellt, dass eine vollständige Verdampfung bzw. Molekülarisierung der Duftstoffe innerhalb sehr kurzer Zeit erfolgt.

Aufgabe der vorliegenden Erfindung ist daher eine Vorrichtung bereitzustellen, die es ermöglicht, Duftstoffe und/oder Duftvernichter unter Vermeidung der vorstehend beschriebenen Nachteile des Standes der Technik gezielt an ihren Bestimmungs- und Wirkort auszubringen.
Weitere Aufgabe der vorliegenden Erfindung ist daher außerdem ein Verfahren zur Erzeugung von Düften und/oder ein Verfahren zur Vernichtung von Düften aus in einem flüssigen Medium enthaltenen Duftstoffen bzw. Duftvernichtern anzugeben, das die vorstehend beschriebenen Nachteile des Standes der Technik vermeidet und insbesondere geeignet ist, verschiedene Düfte zu erzeugen.
Dabei ist insbesondere Aufgabe der vorliegenden Erfindung, geeignete Mittel bereit zu stellen, die Größe der Tropfen zu verringern und außerdem einen schnellen Übergang in die Gasphase zu erreichen, so dass ein Transfer von Duftstoffen innerhalb der zu schaffenden Systeme weitestgehend ausgeschlossen werden kann, und Duftverändungen und Wirkungseinschränkungen ebenfalls weitestgehend ausgeschlossen werden können. Weitere Aufgabe der vorliegenden Erfindung ist außerdem, eine für die Durchführung des Verfahrens geeignete Vorrichtung anzugeben.

Die vorstehenden Aufgaben der vorliegenden Erfindung werden mit den Merkmalen der Ansprüche gelöst.

Vorteilhafte Ausführungen der vorliegenden Erfindung sind in der nachfolgenden Beschreibung erwähnt, die von schematischen Zeichnungen begleitet ist. Hierzu zeigt:
Fig. 1 eine schematische Darstellung der Verfahrensschritte eines ersten erfindungsgemäßen Verfahrens zur Erzeugung von Düften und/oder zur Vernichtung von Düften, bei dem geeigneter Weise eine erste erfindungsgemäße Vorrichtung zur Erzeugung von Düften und/oder Vernichtung von Düften verwendet wird;
Fig. 2 eine vorteilhafte Ausführung des Verfahrens nach Fig.1;
Fig. 3 eine Abwandlung des ersten erfindungsgemäßen Verfahrens bei dem geeigneter Weise eine zweite erfindungsgemäße Vorrichtung zur Erzeugung von Düften und/oder Vernichtung von Düften verwendet wird;
Fig. 4 eine vorteilhafte Ausführung des Verfahrens nach Fig.3;
Fig. 5 eine schematische Darstellung einer ersten erfindungsgemäßen Düse, die geeigneter Weise in den Verfahren nach Fig. 1 und 2, und Fig. 3 und 4 verwendet wird;
Fig. 6 eine schematische Darstellung einer zweiten erfindungsgemäßen Düse, die geeigneter Weise in den Verfahren von Fig. 1 und 2, und Fig. 3 und 4 verwendet wird;
Fig. 7a und 7b eine schematische Darstellung eines erfindungsgemäßen mikroelektromechanischen Bauteils, das im wesentlichen aus zwei oder drei Komponenten besteht, von denen eine Komponente eine Düse gemäß Fig. 5 oder 6 ist;
Fig. 8a eine schematische Darstellung einer ersten erfindungsgemäßen Anordnung zur Erzeugung von Düften und/oder Vernichtung von Düften, die geeigneter Weise in dem Verfahren nach Fig. 1 verwendet wird; und
   Fig. 8b eine vorteilhafte Ausführung der erfindungsgemäßen Anordnung von Fig. 8b, die geeigneter Weise bei dem erfindungsgemäßen Verfahren gemäß Fig. 2 verwendet wird;
Fig. 9a einen Längsschnitt durch einen Teilbereich der Anordnung von Fig. 8a und 8b, und
   Fig. 9b einen Querschnitt durch die Anordnung von Fig. 8 entlang der Linie A - A von Fig. 9a;
Fig. 10a einen Längsschnitt durch eine Abwandlung der Anordnung von Fig. 8, und
   Fig. 10b einen Querschnitt durch eine Abwandlung der Anordnung von Fig. 8 entlang der Linie A - A von Figur 10a; und
Fig. 11 a eine schematische Darstellung einer zweiten erfindungsgemäßen Anordnung zur Erzeugung von Düften und/oder Vernichtung von Düften, die geeigneter Weise bei dem Verfahren gemäß Fig. 3 verwendet wird, und
   Fig. 11b eine vorteilhafte Ausführung der erfindungsgemäßen Anordnung von Fig. 11a, die geeigneter Weise bei dem Verfahren gemäß Fig. 4 verwendet wird;

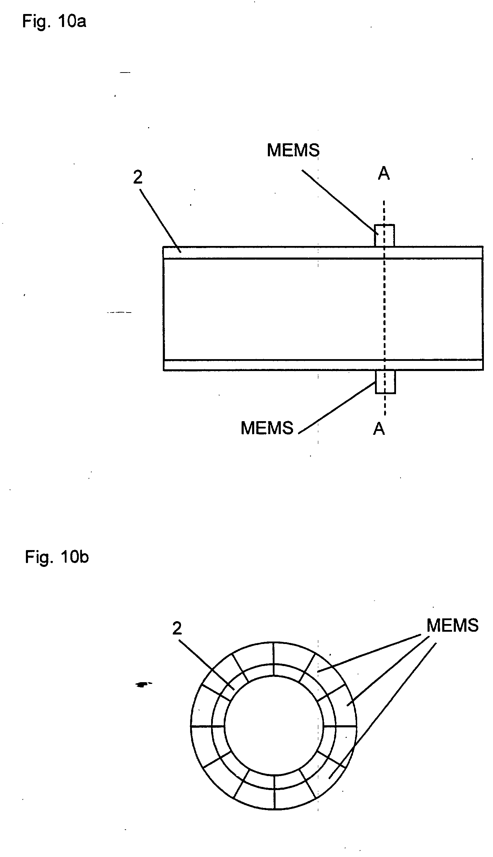

Fig. 10b einen Schnitt durch die Anordnung von Fig. 10a entlang der Linie A - A;
   Fig. 10c einen Schnitt durch eine Abwandlung der Anordnung von Fig. 10a entlang der Linie A - A von Fig. 10a.

Die vorliegenden Erfindung wird nachfolgend anhand von Ausführungsbeispielen beschrieben.

Hierzu sei einleitend gesagt, daß neben Duftstoffen mit dem erfindungsgemäßen Verfahren und der erfindungsgemäßen Anordnung auch vorteilhaft Substanzen ausgebracht werden können, mit denen Düfte/Gerüche gebunden werden können. Diese als Kladrate bekannte Substanzen binden genau Düfte, die an die Stellen der Duftmoleküle, die in unserer Nase binden, und maskieren damit den Duft. Da die Vernebelungseinheit auch diese Stoffe vorteilhaft vemebelt, ergeben sich damit für eine Duftmodulierung in Raum durch ein erfindungsgemäß aufgebautes Gerät besondere Möglichkeiten. Im folgenden ist dieser Umstand gemeint, wenn von Duftvernichtung gesprochen wird.

Daher sei einleitend gesagt, dass wenn nachfolgend ein erfindungsgemäßes Verfahren oder eine erfindungsgemäße Anordnung zur Erzeugung von Düften beschrieben wird, dann soll damit immer auch einschließend ein Verfahren bzw. eine Anordnung zur Vernichtung von Düften gemeint sein.

Grundgedanke der vorliegenden Erfindung ist ein eine Vorrichtung und ein Verfahren zur Erzeugung von Düften aus in einem flüssigen Medium enthaltenen Duftstoffen oder flüssigen Duftstoffen bereitzustellen, bei dem aus dem flüssigen Medium und den Duftstoffen innerhalb einer vorbestimmten Zeit eine Vielzahl kleinster Tropfen mit vorbestimmter maximaler Größe gebildet wird, so dass die minimale Größe in der Größenordnung der Moleküle der Duftstoffe liegt, und die vorbestimmte Zeit derart bemessen ist, dass eine Interaktion der Tropfen in der Luft nicht erfolgt und außerdem eine direkte fluide Benetzung von Oberflächen in der Nähe der Tropfen ausgeschlossen ist.

Bei dem erfindungsgemäßen Verfahren zur Erzeugung von Düften aus in einem flüssigen Medium enthaltenen Duftstoffen oder flüssigen Duftstoffen wird erfindungsgemäß aus dem flüssigen Medium und den Duftstoffen innerhalb einer vorbestimmten Zeit eine Vielzahl kleinster Tropfen mit vorbestimmter Größe mittels Kollision des flüssigen Mediums oder der flüssigen Duftstoffe gebildet, so dass eine Aerosolwolke bereitgestellt ist. Nach einer vorteilhaften Ausführung der vorliegenden Erfindung wird zusätzlich eine weitere Diffusion des Aerosols der Aerosolwolke mittels Energieeinwirkung auf die Aerosolwolke während ihrer Entstehung und/oder kurz nach ihrer Entstehung erzeugt, so dass die Tropfengröße des Aerosols weiter verkleinert wird und dadurch eine direkte fluide Benetzung von Oberflächen in der Nähe der Tropfen vermieden ist, wobei außerdem eine direkte Interaktion der Tropfen vermieden ist und auch eine direkte fluide Benetzung von Oberflächen auch in größerer Entfernung vermieden ist. Die auf diese Weise erzeugte Aerosolwolke wird daraufhin dynamisiert, d. h. zu ihrem Wirkort befördert.

Dabei geht die vorliegende Erfindung außerdem von der Überlegung aus, Duftstoffe mittels Bereitstellung geeigneter Mittel zur Kollision des flüssigen Mediums zu erzeugen, so dass kleinste Tropfengrößen in sehr kleiner Zeit gebildet werden. Dies kann sehr vorteilhaft zunächst durch Selbstkollision des flüssigen Mediums geschehen, aber auch zunächst vorteilhaft durch Kollision des flüssigen Mediums mit einem zweiten flüssigen Medium geschehen, das ebenfalls Duftstoffe enthalten kann, und außerdem kann dies zunächst mittels Kollision des flüssigen Mediums mit einem gasförmigen Medium geschehen. Schon während der Kollision und/oder kurz nach der Bildung der mittels der Kollision erzeugten Aerosolwolke wird nach einer vorteilhaften Ausführung der vorliegenden Erfindung außerdem der Aerosolwolke externe Energie mit einer Energiedichte zugeführt, die geeignet ist, die Größe der die Aerosolwolke bildenden Tropfen weiter zu verringern. Außerdem wird mit der Energiezuführung erreicht, dass die Verdampfung des flüssigen Mediums der Umgebung keine Energie entziehen kann, so dass sicher gestellt ist, dass die Umgebung nicht abkühlt. Mit den erfindungsgemäßen Verfahren werden vorteilhaft ausgehend von Tropfengrößen im Bereich von 3 bis 5 um eine Molekularisierung der Duftstoffe gebildet. Hierdurch wird erreicht, dass zwar Duftmoleküle aber keine Tropfen an Oberflächen niederschlagen können und sich daher nicht nachteilig auf die Beduftung und auf eine erneute Beduftung mit anderen Duftstoffen auswirken. An Oberflächen niedergeschlagene Duftmoleküle sind nämlich anders als niedergeschlagene Tropfen für die Erzeugung von Düften und auch für eine erneute Erzeugung anderer Düfte unschädlich.

Nach einer vorteilhaften Ausbildung des erfindungsgemäßen Verfahrens werden Mittel bereitgestellt, die eine auch hochfrequente Steuerung des Arbeitsbetriebs des Verfahrens gestatten.

Weiterer grundlegender Gedanke der vorliegenden Erfindung ist eine zur Durchführung des obigen Verfahrens geeignete Vorrichtung bereitzustellen, die wenigstens eine geeignet strukturierte Düse aufweist, deren Öffnung in einen Reaktionsraum gerichtet ist, der größer als die Düse ist, wobei an dem Reaktionsraum außerdem ein Mittel zum Transport des Aerosols aus dem Reaktionsraum zu dessen Bestimmungs- und Wirkort bereitgestellt ist.
Der Reaktionsraum ist dabei erfindungsgemäß selbst als Düse ausgebildet, wobei die von der oben beschriebenen ersten kleineren Düse im Innenraum der von dem Reaktionsraum gebildeten zweiten Düse gebildete Aerosolwerke von den Mitteln zum Transport aus der zweiten Düse zu ihrem Bestimmungs- und Wirkort gebracht werden.
Nach einer vorteilhaften Ausführung der vorliegenden Erfindung sind an dem Reaktionsraum außerdem geeignete Mittel angeordnet, um eine weitere Diffusion des Aerosols nach dem Düsenaustritt mittels Bereitstellung von mechanischer und/oder elektromagnetischer Energie zu erzeugen.

Mit dem oben genannten erfindungsgemäßen Verfahren und der erfindungsgemäßen Vorrichtung ist es möglich, Duftstoffe auch ohne Temperaturerhöhung und die damit verbundenen oben genannten Nachteile in kleinsten Teilchengrößen abzuscheiden, so daß die Duftstoffe an ihrem Bestimmungs- und Wirkort zum Einsatz kommen. Dabei wird außerdem erreicht, dass die Düsenöffnungen sehr klein ausgebildet werden können, so dass ein Eintrocknen der Flüssigkeit an den Düsenöffnungen vermieden wird. Außerdem wird mit der vorliegenden Erfindung erreicht, dass die Duftstoffe in derart kleinen in der Größenordnung der Moleküle der Duftstoffe liegenden Tropfengrößen abgeschieden werden, so dass insbesondere eine fluide Benetzung von Oberflächen in der Nähe einer abgeschiedenen Duftstoffwolke vermieden ist. Die erfindungsgemäße Vorrichtung und das erfindungsgemäße Verfahren sind daher besonders geeignet, auch verschiedene Duftstoffe einander abwechselnd und aufeinanderfolgend abzuscheiden, oder verschiedene Duftstoffe miteinander zu vermischen, und/oder Duftstoffe und auch Duftvernichter einander abwechselnd abzuscheiden, da es zu keinen Störeffekten aufgrund von mit zuvor abgeschiedenen Duftstoffen benetzten Oberflächen insbesondere auch innerhalb des der Düse nachgeschalteten Reaktionsraums kommt.

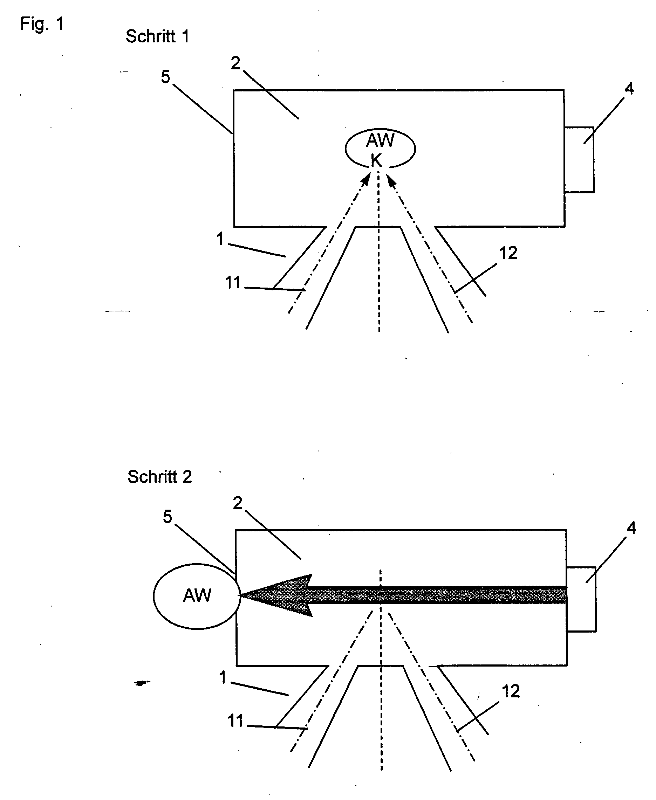

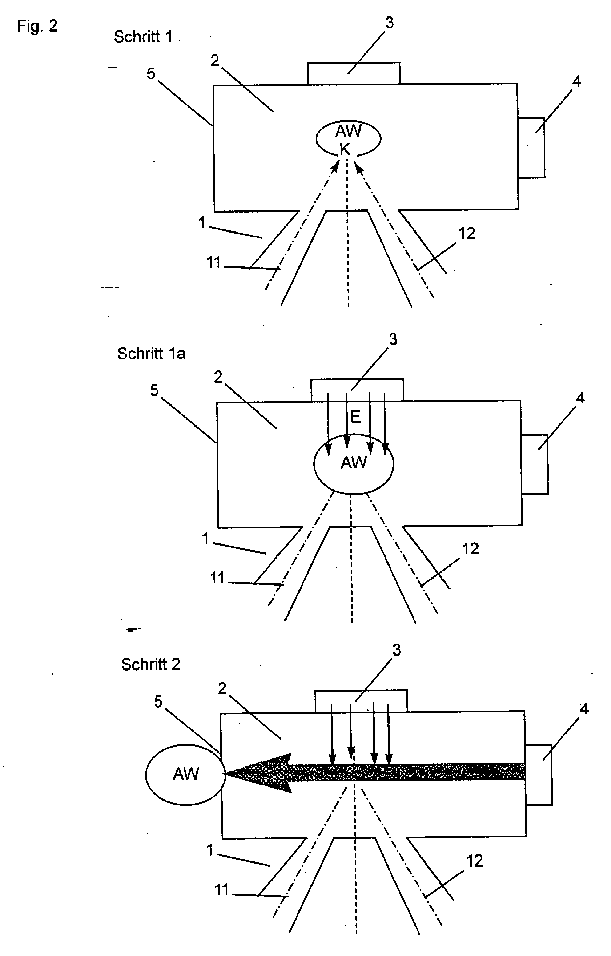

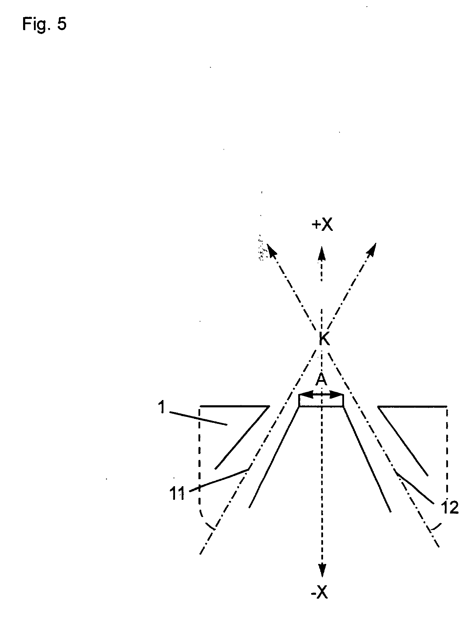

Fig. 1 zeigt eine schematische Darstellung einer ersten Ausführung des erfindungsgemäßen Verfahrens zur Erzeugung von Düften, bei dem in einem ersten Schritt mittels Kollision von in einem flüssigen Medium enthaltenen Duftstoffen in einem Kollisionspunkt K innerhalb eines Reaktionsraums 2 eine Aerosolwolke AW erzeugt wird. Dies geschieht erfindungsgemäß, indem die Flüssigkeit unter hohem Druck aus einer Düse 1 mit wenigstens zwei Öffnungen zur Kollision gebracht wird. Eine geeignete Düse 1 wird nachfolgend anhand von Fig. 5 beschrieben.
In einem erfindungsgemäßen zweiten Schritt 2 wird schließlich die Aerosolwolke AW vollständig aus einer hierfür vorgesehenen Öffnung 5 des Reaktionsraums 2 abgeführt, so dass der Reaktionsraum 2 vollständig von Duftstoffen entleert ist, und außerdem wird die Aerosolwolke AW dabei ihrem Bestimmungs- und Wirkort zugeführt. Hierfür werden ebenfalls geeignete an dem Reaktionsraum 2 angeordnete Mittel 4 zum Transport der Aerosolwolke verwendet.
Nach einer vorteilhaften Ausführung der vorliegenden Erfindung, die schematisch in Fig. 2 schematisch dargestellt ist, wird in einem erfindungsgemäßen weiteren Schritt 1a die in Schritt 1 erzeugte Aerosolwolke AW außerdem unter Energieeinwirkung in dem Reaktionsraum 2 nachbehandelt, wobei ein geeigneter Energiegenerator 3 verwendet wird. Hierdurch werden wie oben gesagt, die in der Aerosolwolke AW enthaltenen Tropfen vorteilhaft weiter zerkleinert und außerdem sichergestellt, dass bei der Verdampfung der Duftstoffe der Umgebung keine Energie entzogen wird. Außerdem wird durch die Energiezuführung E zu der Aerosolwolke AW sichergestellt, dass keine Restflüssigkeitstropfen an der inneren Oberfläche des Reaktionsraumes 2 niederschlagen.

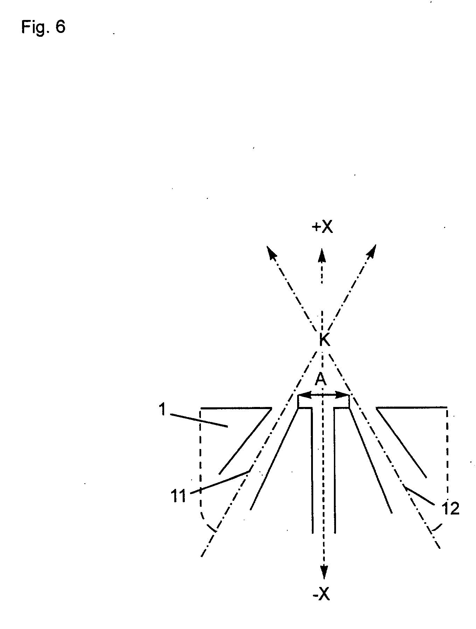

Bei den erfindungsgemäßen Verfahren gemäß Schritt 1 und Schritt 2, und gemäß Schritt 1, Schritt 1a und Schritt 2 nach Fig. 1 und Fig. 2, die oben beschrieben sind, erfolgt der Abtransport der Aerosolwolke AW aus dem Reaktionsraum 2 geeigneter Weise im wesentlichen senkrecht zu dem Austritt der Duftstoffe aus den Düsenöffnungen 11 und 12 der Düse 1. Eine bei dem Verfahren gemäß Fig. 1 und Fig. 2 geeignet verwendbare Düse 1 wird nachfolgend anhand der Beschreibung von Fig. 5 näher erläutert, und für das Verfahren gemäß Fig. 1 und Fig. 2 geeignete Anordnungen werden nachfolgend anhand der Beschreibung von Fig. 6 näher erläutert. Erfindungsgemäß werden bei dem Verfahren nach Fig. 1 und Fig. 2 die Schritte 1 und 2, bzw. die Schritte 1, 1a und 2 synchron geschaltet, wobei Schritt 1 und Schritt 2, und bzw. Schritt 1, Schritt 1a und Schritt 2 vorteilhaft gleichzeitig gestartet werden können, wenn ein entsprechend träge ansteuerbares Mittel 4 zum Transport der Aerosolwolke AW aus dem Reaktionsraum 2 verwendet wird. Außerdem kann vorteilhaft nach dem Start von Schritt 1 bzw. nach dem gleichzeitigen Start von Schritt 1 und Schritt 1a Schritt 2 mit einer vorbestimmten geringen Verzögerung gestartet werden, wenn eine längere Verweildauer der Aerosolwolke AW in dem Reaktionsraum 2 wünschenswert ist. Um eine gleichmäßige Energiebehandlung der Duftstoffe AW in dem Reaktionsraum 2 zu gewährleisten, kann es außerdem vorteilhaft sein, dass Schritt 2 gestartet wird, nachdem Schritt 1 und/oder Schritt 1a beendet sind.

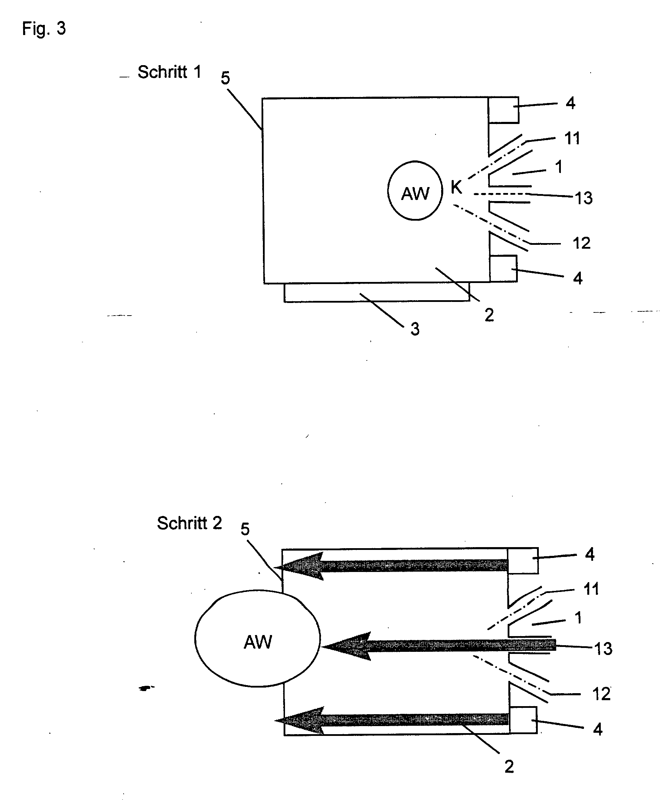

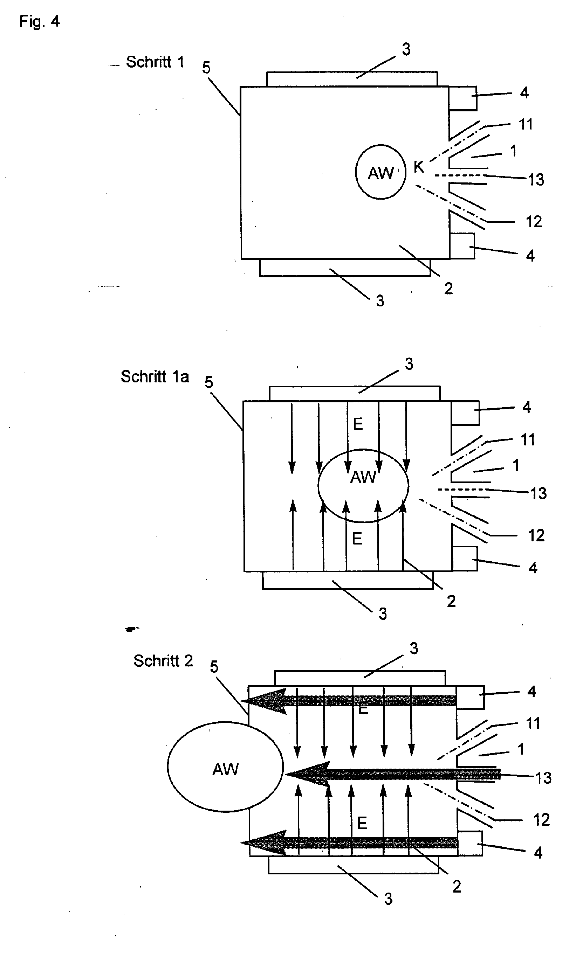

Nachfolgend wird anhand von Fig. 3 und Fig. 4 eine zweite vorteilhafte Ausführung der vorliegenden Erfindung beschrieben, die eine Abwandlung der oben anhand von Fig. 1 und Fig. 2 beschriebenen erfindungsgemäßen Verfahren ist, und das im wesentlichen ebenfalls aus den oben beschriebenen Verfahrensschritten Schritt 1 und Schritt 2, bzw. Schritt 1, Schritt 1a und Schritt 2 besteht, wobei anders als bei dem Verfahren von Fig. 1 und Fig. 2 das verwendete Mittel zur Kollision 1, das nach einer vorteilhaften Ausführung der vorliegenden Erfindung gemäß Fig. 4 verwendete Mittel zur Energiezuführung 3 und das verwendete Mittel zum Transport 4 in einer Geometrie an dem Reaktionsraum 2 angeordnet werden, so dass die Düse 1 gegenüber der Öffnung 5 des Reaktionsraums angeordnet ist, und dass der Abtransport der Aerosolwolke AW aus dem Reaktionsraum 2 mit den hierfür vorgesehenen Mitteln 4 im wesentlichen parallel zu dem Düsenaustritt der Duftstoffe aus der Düse 1 erfolgt. Diese geometrische Anordnung ist besonders vorteilhaft, wenn nur wenig verschiedene Duftstoffe aus verschiedenen Düsen 1 in den Reaktionsraum 2 eingebracht werden sollen. Es ist im übrigen klar, dass die beiden Verfahren nach Fig. 1 und Fig. 2, und Fig. 3 und Fig. 4 auch vorteilhaft miteinander kombiniert werden können.

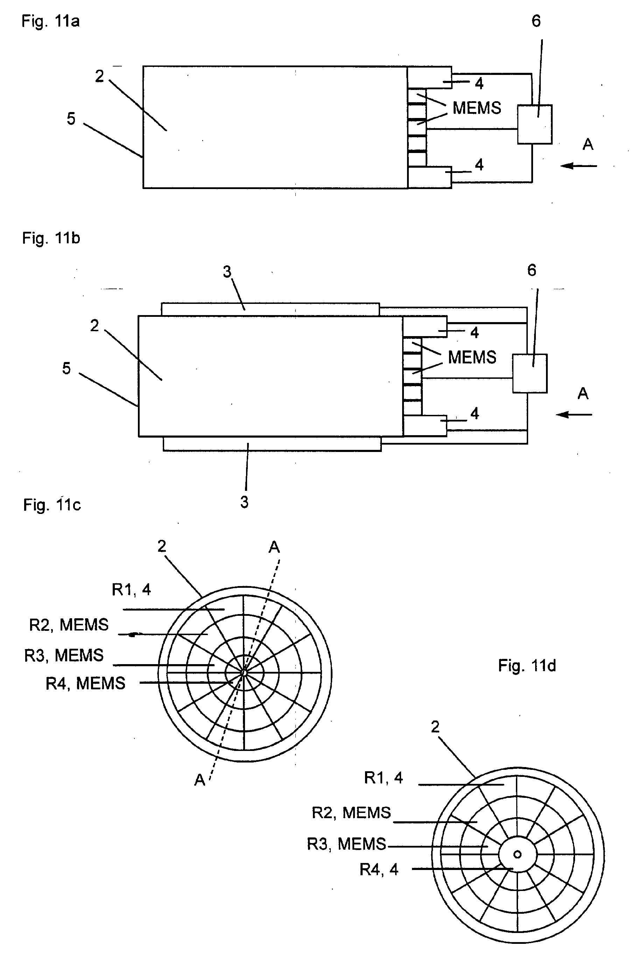

Bei dem Verfahren gemäß Fig. 3 und gemäß Fig. 4 wird vorteilhaft eine Düse verwendet, die nachfolgend anhand der Beschreibung von Fig. 6 näher erläutert wird, und eine für das Verfahren gemäß Fig. 1 und gemäß Fig. 2 geeignete Anordnung wird nachfolgend anhand der Beschreibung von Fig. 11 näher erläutert. Es ist auch klar, dass jedoch an Stelle der Düse gemäß Fig. 6 auch die Düse gemäß Fig. 5 verwendet werden kann, was ebenso umgekehrt für die Verfahren gemäß Fig. 1 und Fig. 2 gilt.

Fig. 5 zeigt eine schematische Darstellung einer Düse 1, die vorteilhaft bei dem erfindungsgemäßen Verfahren gemäß Fig. 1 bis Fig. 4 verwendet wird, bei denen wenigstens zwei Flüssigkeitsstrahlen 11, 12 unter hohem Druck auf einen Kollisionspunkt K gerichtet werden. Dabei ist die Höhe des Druckes und die Strahlendicke derart bemessen, dass es bei der Kollision in dem Punkt K zu einer Feinstzerstäubung des flüssigen Mediums und der Tropfengröße in Molekülgröße der in dem flüssigen Medium enthaltenen Duftstoffe kommt. In der Zeichnung schließen die beiden Flüssigkeitsstrahlen einen Winkel von beispielsweise ungefähr 60 Grad ein. Es ist klar, dass der Winkel in einem Bereich variiert werden kann, solange sichergestellt ist, dass einerseits bei der Kollision der beiden Flüssigkeitsstrahlen überwiegend Tropfen mit kinetischer Energie in positiver X-Richtung gebildet werden, sodaß Rückschlageffekte der Tropfen weitestgehend vermieden werden, und andererseits solange der Winkel zwischen den beiden Strahlrichtungen einen Betrag hat, der eine hinreichende Kollision der Strahlen gewährleistet. Mit anderen Worten nimmt mit zunehmendem Winkel zwar das Kollisionspotential wünschenswert zu, aber gleichzeitig nehmen Rückschlageffekte auch aufgrund der Verlagerung des Kollisionspunktes K in negativer X-Richtung zu. Erfindungsgemäß liegen geeignete Winkel daher in einem Bereich zwischen 30° und 120°, vorteilhaft zwischen 45° und 90°).

Bei der Verwendung der Düse 1 gemäß Fig. 5 in den erfindungsgemäßen Verfahren gemäß Fig. 1 bis Fig. 4 ist daher sichergestellt, dass die Oberfläche des kollidierenden Mediums nicht eine Oberfläche des Reaktionsraumes benetzt, und dass die zunächst bei der Kollision gebildeten Tropfen so klein sind, dass sie unter der Energieeinwirkung E in dem Reaktionsraum 2 innerhalb sehr kurzer Zeit unmittelbar nach der Kollision zu Molekülen verdampfen. Hierdurch wird erreicht, dass lediglich Duftmoleküle, aber keine Tropfen an der inneren Wand des Reaktionsraumes 2 und an weiter entfernten Oberflächen niederschlagen können und sich daher nicht nachteilig auf die Beduftung und auch erneute Beduftung mit anderen Duftstoffen auswirken.

Die oben anhand von Fig. 1 und Fig. 2, und Fig. 3 und Fig. 4 beschriebenen erfindungsgemäßen Verfahren und die erfindungsgemäße Düse gemäß Fig. 5 sind insbesondere zur Selbstkollision eines flüssigen Mediums und/oder zur Kollision zweier flüssiger Medien mit verschiedenen Duftstoffen und/oder einem flüssigen Medium, das einen Duftstoff enthält mit einem neutralen flüssigen Medium und/oder eines flüssigen Mediums mit einem gasförmigen Medium geeignet.

Fig. 6 zeigt eine erfindungsgemäße Abwandlung der Düse gemäß Fig. 5, wobei zusätzlich zu den beiden Strahlengängen 11 und 12 ein dritter Strahlengang 13 ebenfalls auf den Kollisionspunkt K gerichtet ist. Dabei ist der Strahl 13 geeigneter Weise in der Ebene ausgebildet, die von den beiden Strahlen 11 und 12 vorgegeben ist, und außerdem geeigneter Weise entlang der Winkelhalbierenden des Winkels zwischen den beiden Kanälen 11 und 12 ausgerichtet. Für den Betrag des Winkels zwischen den Kanälen 11 und 12 und für den Abstand A, der in einem Winkel aufeinander gerichteten Strahlen 11 und 12 gilt auch hier das diesbezüglich oben zur Fig. 5 gesagte, mit dem Unterschied, dass aufgrund des Strahles 13 mehr kinetische Energie in positiver X-Richtung zur Verfügung steht, und der optimale Winkel dementsprechend größer als bei der Ausführung von Fig. 5 gewählt werden kann. Für den Abstand A der Öffnungen der beiden äußeren Kanäle 11 und 12, die hier ebenso wie bei der oben beschriebenen Ausführung der Düse 1 die Richtung der beiden äußeren Strahlen 11 und 12 vorgeben, gilt bei der hier beschriebenen zweiten Ausführung der erfindungsgemäßen Düse 1 auf gleiche Weise das oben zur ersten Ausführung der erfindungsgemäßen Düse 1 gesagte.

Die zweite Ausführung der erfindungsgemäßen Düse 1 ist besonders geeignet, ein flüssiges Medium, das einen Duftstoff enthält, mittels Selbstkollision über die beiden Flüssigkeitsstrahlen 11 und 12 und außerdem mittels einem dritten Flüssigkeitsstrahl 13 zu zerstäuben und/oder außerdem unter Kollision mit einem zweiten flüssigen Medium mit einem zweiten Duftstoff und/oder einem gasförmigen Medium, das entlang dem Strahl 13 gerichtet ist, zu zerstäuben. Erfindungsgemäß können wahlweise gleiche und/oder verschiedene flüssige und/oder gasförmige Medien, die wahlweise gleiche und/oder unterschiedliche Duftstoffe enthalten können entlang der Strahlen 11 und 12 und 13 in dem Punkt K zur Kollision gebracht werden.

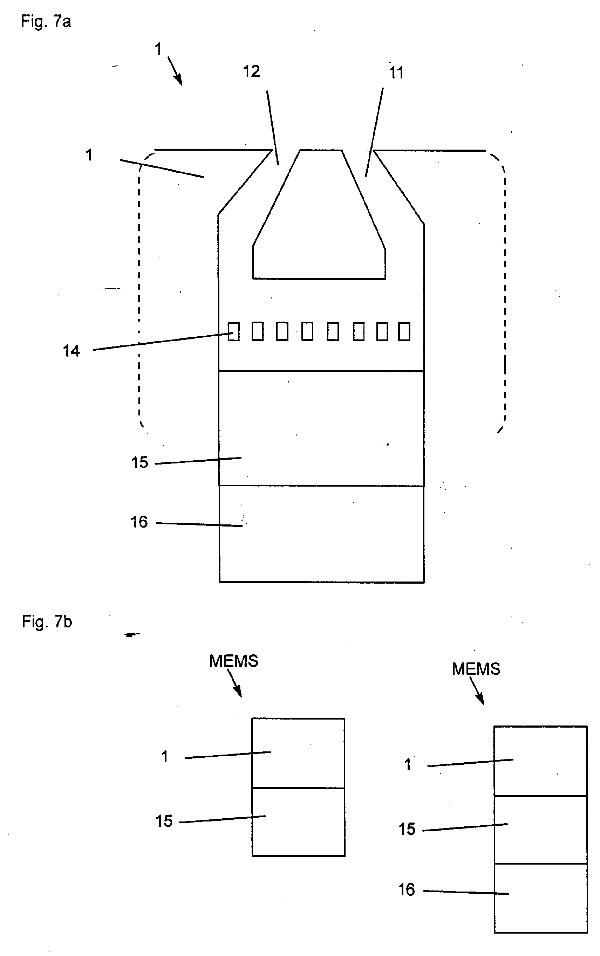

Fig. 7a zeigt einen vergrößerten Ausschnitt der Öffnung der erfindungsgemäßen Düse 1 gemäß Fig. 5, wobei außerdem der weitere Verlauf der Kanäle 11 und 12 aufgezeigt ist, die in einen stark vergrößerten Kanalbereich münden, in dem geeigneter Weise Filterstrukturen 14 ausgebildet sein können, die ein Verstopfen der Kanäle 11 und 12 insbesondere an ihren Öffnungen durch etwaige Verunreinigungen in dem flüssigen Medium schützen. Eine Erweiterung des Querschnitts der Kanäle ist besonders vorteilhaft zur Erzielung eines hohen Austrittsdruckes des flüssigen Mediums. Es ist klar, dass Fig. 7a auf eine Ausführung des erfindungsgemäßen Verfahrens gerichtet ist, die eine Zerstäubung des flüssigen Mediums mittels Selbstkollision des flüssigen Mediums bewirkt.

Der für eine Zerstäubung des flüssigen Mediums benötigte Druck wird geeigneter Weise mittels einem der Düse 1 vorgeschalteten und ebenfalls miniaturisierten Bauteil 15 erzeugt, das geeigneter Weise mit der Düse 1 ein Bauteil sein kann und vorteilhaft mittels einem piezoelektrischen Aktor bereitgestellt wird. Piezoelektrische Aktoren sind als Mittel zur Druckerzeugung besonders geeignet, da sie ohne Wärmeerzeugung den für die Kollision und die Zerstäubung benötigten Druck aufbauen können, miniaturisiert herstellbar sind und hoch frequent schaltbar sind. Dem piezoelektrischen Aktor 15 ist außerdem ein geeignet ausgebildeter flexibler Behälter 16 vorgeschaltet, der als Vorratsbehälter für das flüssige Medium dient, und der geeigneter Weise derart ausgebildet ist, dass ein Nachdringen von Luft bei absinkendem Flüssigkeitsstand ausgeschlossen ist. Erfindungsgemäß sind außerdem Düse 1, Mittel zur Druckausübung 15 und Vorratsbehälter 16 derart ausgebildet, dass ein Eindringen von Luft in das System Düse 1, Mittel zur Druckausübung 15 und Vorratsbehälter 16 ausgeschlossen ist. Wie in Fig. 7b schematisch dargestellt ist, kann geeigneter Weise Düse 1 und Mittel zur Druckausübung 15 mittels einem mikroelektromechanischen Systems als ein miniaturisiertes Bauteil (MEMS) bereitgestellt sein, und es kann auch geeigneter Weise Düse 1, Mittel zur Druckausübung 15 und Vorratsbehälter 16 als ein MEMS ausgebildet sein.

Die oben beschriebenen und in den Figuren 7a und 7b schematisch dargestellten erfindungsgemäßen MEMS wurden beispielhaft anhand einer Einstoff-Düse gemäß der Ausbildung von Fig. 7a, die eine Selbstkollision eines flüssigen Mediums bewirkt, erläutert. Erfindungsgemäß werden für Zweistoff- oder Mehrstoffdüsen der Ausbildung gemäß Fig. 5 und Fig. 6 entsprechend abgewandelte MEMS auf gleiche Weise bereitgestellt.

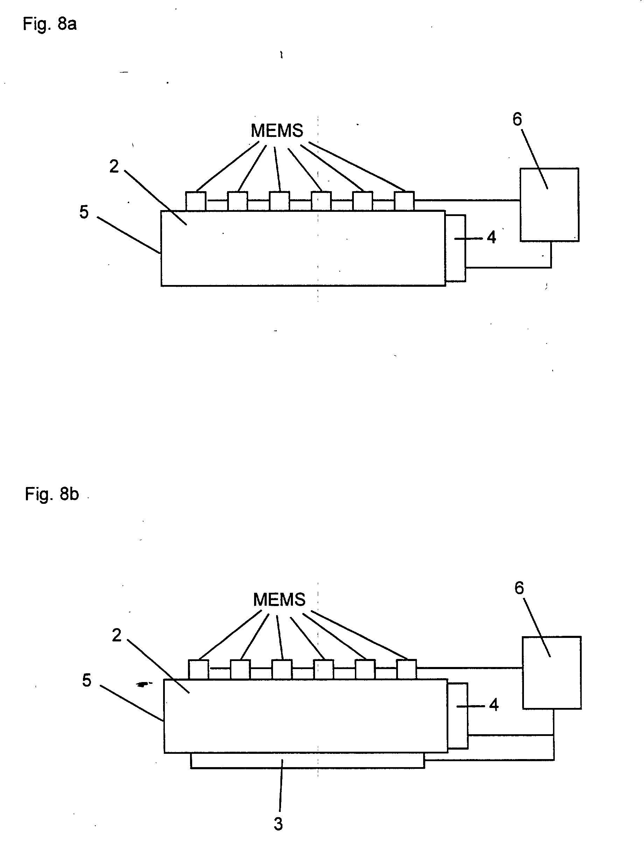

Fig. 8a zeigt eine schematische Darstellung einer erfindungsgemäßen Vorrichtung zur Durchführung des erfindungsgemäßen Verfahrens gemäß Fig. 1, bei der erfindungsgemäß wenigstens ein mikrostrukturiertes MEMS derart an einem Reaktionsraum 2 angeordnet ist, dass die mikrostrukturierte Düse 1 des Bauteils MEMS in den Reaktionsraum 2 gerichtet ist. An dem Reaktionsraum 2 ist außerdem ein Ventilator und/oder Druckluftgenerator 4 angeordnet, der einen Luftstrom zum Transport des Aerosols AW aus der Öffnung 5 des Reaktionsraums 2 bereitstellt. Zur Steuerung der MEMS, des Energiegenerators für die weitere Diffusion 3 und des Mittels zum Transport 4 ist außerdem eine geeignete Elektronik 6 vorgesehen, die die Anordnung gemäß den oben beschriebenen erfindungsgemäßen Verfahren steuert. Der Reaktionsraum 2 ist hierbei erfindungsgemäß als zweite größere Düse 2 ausgebildet, in deren Innenraum mittels der ersten kleineren Düse 1 eine Aerosolowolke erzeugt wird, die aus der zweiten größeren Düse 2 mit geeigneten an der zweiten Düse 2 angeordneten Transportmitteln 4 zu ihrem Bestimmungs- und Wirkort gebracht wird. Diese erfindungsgemäße Düsenstruktur, die aus einer ersten kleinen Düse besteht, die eine Aerosolwolke in dem Inneren einer zweiten Düse erzeugt, ist für das Ausbringen von Duftstoffen besonders vorteilhaft, da Aerosolbildung und Transport des Aerosols vorteilhaft voneinander getrennt sind. Fig. 8b zeigt eine schematische Darstellung einer vorteilhaften Ausführung der erfindungsgemäßen Anordnung gemäß Fig. 8a, bei der an dem Reaktionsraum 2 zur Bereitstellung von mechanischer und/oder elektromagnetischer Energie für die weitere Diffusion des Aerosols AW nach dem Düsenaustritt außerdem ein geeigneter Druckluft- und/oder Ultraschall- und/oder Elektronenstrom- und/oder Mikrowellengenerator angeordnet sind.

Erfindungsgemäß können eine Vielzahl voneinander unabhängiger MEMS zur Ausbringung von gleichen Duftstoffen, verschiedenen Duftstoffen, neutralen Stoffen, oder Duftvernichtern in geeigneter Weise an dem Reaktionsraum 2 angeordnet sein, wobei die MEMS über die Elektronik 6 erfindungsgemäß unabhängig voneinander ansteuerbar sind, sodaß sie zeitgleich und/oder zeitversetzt schaltbar sind.

Der Reaktionsraum 2 hat ein Volumen, das sehr viel größer ist als die Öffnung einer Düse 1 eines Bauteils MEMS und kann gemäß der schematischen Anordnung von Fig. 8a und 8b beispielsweise zylindrisch ausgebildet sein, wobei die MEMS bei der hier beschriebenen Anordnung geeigneter Weise linear am Mantel des Zylinders angeordnet sind, und das Transportmittel 4 gegenüber der Öffnung 5 am Boden des Zylinders angeordnet ist. Der Reaktionsraum 2, der sehr viel größer als eine Düsenöffnung der MEMS ist, ist außerdem in Abhängigkeit von der Struktur und Anordnung der MEMS und den mechanischen, elektromagnetischen und fluidischen Eigenschaften des flüssigen Mediums derart dimensioniert ausgebildet, dass das Aerosol AW an seiner inneren Wand nicht niederschlägt.
Das Mittel 3 zur Erzeugung von Energie für die Nachbehandlung des Aerosols in dem Reaktionsraum 2 bei der vorteilhaften Ausführung gemäß Fig. 8b ist ebenfalls am Mantel des Zylinders angeordnet, und geeigneter Weise als Druckluft- und/oder Ultraschall- und/oder Elektronenstrom- und/oder Mikrowellengenerator mit der Eigenfrequenz des flüssigen Mediums ausgebildet, so dass innerhalb kurzer Zeit die für eine weitere Diffusion des Aerosols in dem Reaktionsraum 2 benötigte Energie bereitgestellt werden kann. Der Reaktionsraum 2 ist außerdem derart auf die Frequenz des Ultraschall- und/oder Elektronenstrom- und/oder Mikrowellengenerators geeignet abgestimmt ausgebildet, daß eine stehende Welle innerhalb des Reaktionsraumes 2 mittels dem Generator 3 bereitgestellt werden kann. Außerdem besteht der Reaktionsraum 2 vorteilhaft aus leitfähigem Material, wobei geeigneter Weise seine innere Wand aus duftinertem und/oder passiviertem Material ausgebildet ist.

Die MEMS können Einstoffdüsen gemäß Fig. 5 und/oder Zweistoffdüsen gemäß Fig. 6 umfassen, wobei die MEMS mit Zweistoffdüsen, die auch Druckluft in den Reaktionsraum 2 einspeisen, so geschaltet sein können, dass bei jeder Aktivierung wenigstens eines der MEMS zugleich auch alle anderen MEMS, die eine Druckluftdüse aufweisen, Druckluft in den Reaktionsraum 2 einspeisen. Hierdurch wird eine Benetzung der inneren Wand des Reaktionsraums 2 weiter verhindert. Außerdem können auch eine Vielzahl von weiteren Bauteilen MEMS zur Ausbringung von Druckluft an geeigneten Positionen an dem Reaktionsraum angeordnet sein, so dass ein Luftstrom über der inneren Wand des Reaktionsraums 2 ausbildbar ist.

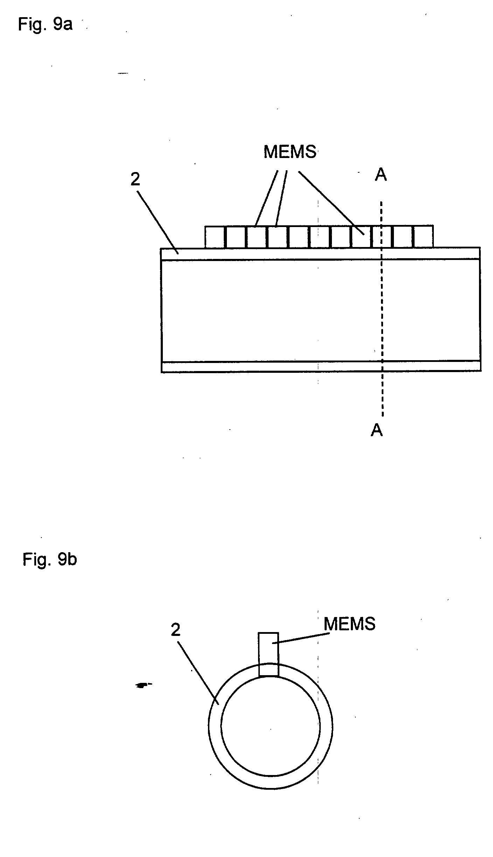

Fig. 9a zeigt eine vorteilhafte lineare Anordnung der MEMS an der Wand des Reaktionsraums 2 im Längsschnitt, und Fig. 9b zeigt einen Querschnitt durch den Reaktionsraum 2 entlang der Linie A - A von Fig. 9a. Eine lineare Anordnung der MEMS ist insbesondere von Vorteil, da sie auf einfache Weise billig hergestellt werden kann, und außerdem da die Vorratsbehälter 16 der Duftstoffe bei dieser Anordnung auf einfache Weise ersetzt bzw. nachbefüllt werden können.

Fig. 10a zeigt eine Abwandlung der erfindungsgemäßen Vorrichtung gemäß Fig. 8, bei der die MEMS zirkular an einem Reaktionsraum 2 angeordnet sind, im Längsschnitt, und Fig. 10b zeigt einen Schnitt durch die Linie A - A von Fig. 10a. Geeigneter Weise sind hier die MEMS in einem Ring angeordnet, der auch als Einheit austauschbar ist. Hierdurch werden ebenfalls Herstellungskosten erniedrigt und außerdem der Betrieb der Vorrichtung erleichtert, und ein einfaches Nachrüsten ermöglicht. Außerdem gestattet diese Anordnung geeignete vorgefertigte Duft-Sets bereitzustellen, die jeweils eine Zusammenstellung geeigneter Düfte in der ringförmigen Anordnung enthält, die jeweils auf einfache Weise in der erfindungsgemäßen Vorrichtung angeordnet werden können. Es ist klar, dass eine erfindungsgemäße Anordnung auch eine Vielzahl derartiger Ringe enthalten kann. Es ist auch klar, dass die MEMS vorteilhaft auch spiralförmig an dem Reaktionsraum 2 angeordnet sein können , und dass die MEMS auch gleichmäßig über die gesamte Oberfläche des Zylindermantels des Reaktionsraums 2 angeordnet sein können.

Fig. 11a, b, c und d zeigen schematische Darstellungen einer erfindungsgemäßen Vorrichtung, die zur Durchführung des erfindungsgemäßen Verfahrens gemäß Fig. 2 geeignet ist. Die erfindungsgemäßen Vorrichtungen von Fig. 11 entsprechen im wesentlichen den oben beschriebenen Vorrichtungen von Fig. 8a und b, mit dem Unterschied, dass die MEMS gegenüber der Öffnung 5 des Reaktionsraumes 2 am Boden eines ebenfalls beispielhaft zylinderförmigen Reaktionsraums 2 angeordnet sind, wobei die MEMS sich die Bodenfläche mit den Transportmitteln 4 zum Transport der Aerosolwolke aus dem Reaktionsraum 2 teilen. Die übrige Anordnung entspricht im wesentlichen der oben beschriebenen Vorrichtung gemäß Fig. 8, weshalb hier auf die diesbezügliche Beschreibung verwiesen wird.
Der Reaktionsraum 2 ist hierbei ebenfalls erfindungsgemäß als zweite größere Düse 2 ausgebildet, in deren Innenraum mittels der ersten kleineren Düse 1 eine Aerosolowolke erzeugt wird, die aus der zweiten größeren Düse 2 mit geeigneten an der zweiten Düse 2 angeordneten Transportmitteln 4 zu ihrem Bestimmungs- und Wirkort gebracht wird. Diese erfindungsgemäße Düsenstruktur, die aus einer ersten kleinen Düse besteht, die eine Aerosolwolke in dem Inneren einer zweiten Düse erzeugt, ist für das Ausbringen von Duftstoffen besonders vorteilhaft, da Aerosolbildung und Transport des Aerosols vorteilhaft voneinander getrennt sind.

Die MEMS der Vorrichtung gemäß Fig. 11 können ebenfalls als Einstoffdüse und/oder Zweistoffdüse gemäß Fig. 5 bzw. Fig. 6 ausgebildet sein, wobei deren Ansteuerung der oben beschriebenen Ansteuerung der MEMS der Anordnung von Fig. 8 entspricht, so dass hier ebenfalls darauf verwiesen wird. Bei der erfindungsgemäßen Anordnung von Fig. 11 kann besonders vorteilhaft ein MEMS mit einer Zweistoffdüse deren einer Kanal ein Druckluftkanal ist, verwendet werden, wobei bei geeigneter Ansteuerung der Druckluft der MEMS auch auf das zusätzliche Mittel 4 zum Transport der Aerosolwolke aus dem Reaktionsraum 2 verzichtet werden kann. Es ist jedoch auch vorteilhaft, zusätzlich zu den MEMSn geeignete Mittel 4 zum Transport der Aerosolwolke aus dem Reaktionsraum 2 bereitzustellen, die geeigneter Weise an einem äußeren Ring R1,4 des Zylinderbodens angeordnet sind, und auf einfache Weise mittels geeigneter Löcher in dem Zylinderboden und einem einzigen hinter dem MEMSn angeordneten Ventilator bereitgestellt werden können. Durch diese Anordnung wird vorteilhaft ein Niederschlag der Duftstoffe an der inneren Zylinderwand weiter verhindert. Die MEMS sind geeigneter Weise gleichmäßig über den Zylinderboden verteilt, wobei neben den Transportmitteln 4 am Rand des Bodens auch noch weitere Transportmittel 4 beispielsweise in der Mitte des Boden R4, 4 angeordnet sein können, wie in Fig. 11c und 11d dargestellt ist.

Mit dem oben beschriebenen erfindungsgemäßen Verfahren gemäß Fig. 1 und 2, und Fig. 3 und Fig. 4 und den erfindungsgemäßen Anordnungen gemäß Fig. 8 und 11 kann auf einfache Weise die Ausbringungsmenge von Duftstoffen pro MEMS pro Arbeitsintervall in einem weiten Bereich zwischen 1 Pikogramm bis 100 µl eingestellt werden. Außerdem ist die Zeitdauer des Arbeitsintervalls der Duftausbringung vergleichsweise in einer sehr großen Bandbreite regelbar, wobei die Zeitfolge zwischen zwei Duftausbringungen sehr kurz oder Null sein kann, so dass auch Düfte gezielt mischbar sind. Hierbei erfolgt die Steuerung über elektronische Impulse oder elektronische Datenverarbeitungsprogramme und bewirkt eine vorbestimmte Menge und/oder Zusammensetzung eines Duftes, wobei dessen Ausbringung synchron zu anderen Ereignissen erfolgen kann, eine Zeitvorwahl aufweisen kann und auch periodisch einstellbar sein kann. Die oben beschriebenen Vorrichtungen gemäß Fig. 8 und 11 können außerdem geeignete Sensoren enthalten, die die elektronische Steuerung 6 bei deren Detektion von beispielsweise optischen, akustischen, thermischen, etc. Signalen auf eine vorbestimmte Weise veranlassen, eine vorbestimmte Duftausbringung zu aktivieren, wobei die Steuerung auch mit einer Rückkopplungsfunktion versehen sein kann, die ebenfalls mit einem Sensor zusammenwirken kann.

Die erfindungsgemäße Vorrichtung ist besonders geeignet für tragbare und/oder fest installierbare Geräte, die in Büro, Haushalt, Arbeitsplatz oder für Promotionszwecke, z. B. im Handel zu Verbesserung der Raumluft durch Ausbringung von Duftstoffen und anderen Stoffen eingesetzt werden können, wobei die Ausbringung synchron zu anderen medialen Ereignissen erfolgen kann. Die erfindungsgemäße Vorrichtung ist außerdem vorteilhaft derart ausgebildet, dass sie mit anderen elektrischen oder elektronischen Geräten gekoppelt werden kann oder dass sie mit anderen elektrischen oder elektronischen Geräten kommunizieren kann. Beispielsweise kann sie in Funkverbindung mit einem Filmvorführgerät stehen. Die Vorrichtung ist außerdem vorteilhaft über andere elektrische oder elektronische Geräte steuerbar ausgebildet, wobei sie auch in andere elektrische oder elektronische Geräte integriert werden kann und/oder mit diesen Geräten eine Einheit bilden kann. Hierfür kommen bevorzugt Geräte der Informations- und Kommunikationstechnik in Frage, wie beispielsweise Fernseher, Radio, Telefon, Mobiltelefon, Computer, multifunktionale Informations- und Kommunikationsgeräte, etc., und außerdem Geräte, die in der Hygiene-, Umwelt-, Haus-, Fahrzeug- und Klimatechnik eingesetzt werden, wie insbesondere Desinfektionsgeräte, Klimageräte, Verdunstungsgeräte, Zerstäubungsgeräte und Befeuchtungsgeräte.

Mittels den erfindungsgemäßen Verfahren und den erfindungsgemäßen Anordnungen werden die eingangs genannten Nachteile des Standes der Technik vermieden, wie aus der obigen detaillierten Beschreibung der vorteilhaften Ausführungen der vorliegenden Erfindung hervorgeht.

## Patentansprüche

1. Verfahren zum Erzeugen und/oder zur Neutralisierung von Düften mittels in einem flüssigen Medium enthaltenen Duftstoffen und/oder Duftvernichtern oder flüssigen Duftstoffen und/oder Duftvernichtern mit den Schritten:
Schritt 1:
Selbstkollision der Duftstofe und/oder Duftvernichter und/oder Kollision der Duftstoffe und/oder Duftvernichter mit Druckluft und/oder Kollision der Duftstoffe und/oder Duftvernichter mit einer duftneutralen Flüssigkeit mittels Ausbringen der Duftstoffe und/oder Duftvernichter und/oder Druckluft und/oder duftneutralen Flüssigkeit unter hohem Druck aus einer geeignet strukturierten miniaturisierten Düse (10) in einen der Düse (10) nachgeschalteten Reaktionsraum (2), so daß ein Aerosol (AW) erzeugt wird;
Schritt 2:
Bereitstellung von Mitteln (4) zum Transport des Aerosols (AW) aus dem Reaktionsraum (2) und Transport des Aerosols (AW) aus dem Reaktionsraum (2).

2. Verfahren nach Anspruch 1, **dadurch gekennzeichnet, daß** es außerdem umfaßt:
Schritt 1a:
Bereitstellung von Energie (E) in dem nachgeschalteten Reaktionsraum (2), so daß eine weitere Diffusion des Aerosols (AW) nach dem Düsenaustritt erfolgt;

3. Verfahren nach Anspruch 1 und 2, wobei Mittel (MEMS, 3, 4, 6) bereitgestellt sind, die eine auch hochfrequente Steuerung von Schritt 1 und Schritt 2 oder Schritt 1, Schritt 1a und Schritt 2 gestatten.

4. Verfahren nach einem der Ansprüche 1 bis 3, wobei die Düse (1) derart ausgebildet ist, daß in das Innere der Düse (1) keine Luft gelangt.

5. Verfahren nach einem der vorstehenden Ansprüche 1 bis 4, wobei die Mittel (4) in Schritt 2 mittels Druckluftquelle und/oder Ventilator bereitgestellt werden, und wobei ein Luftstrom zum Transport des Aerosols (AW) aus dem Reaktionsraum (2) erzeugt wird.

6. Verfahren nach einem der vorstehenden Ansprüche 2 bis 5, wobei die Energie (E) in Schritt 2 mittels Druckluftquelle und/oder Ventilator und/oder Elektronenstrom und/oder Mikrowellen und/oder akustischen Wellen bereitgestellt wird.

7. Verfahren nach einem der Ansprüche 1 bis 6, wobei
Schritt 1 und Schritt 2 synchron geschaltet werden, und wobei Schritt 1 und
Schritt 2 gleichzeitig gestartet werden, und/oder Schritt 2 mit einer vorbestimmten geringen Verzögerung gestartet wird, und/oder Schritt 2 gestartet wird nachdem Schritt 1 beendet ist.

8. Verfahren nach einem der vorstehenden Ansprüche 2 bis 7, wobei
Schritt 1 und Schritt 1a und Schritt 2 synchron geschaltet werden, und wobei
Schritt 1 und Schritt 1a und Schritt 2 gleichzeitig gestartet werden, und/oder
Schritt 1 und Schritt 1a gleichzeitig gestartet wird und Schritt 2 mit einer
vorbestimmten geringen Verzögerung gestartet wird, und/oder
Schritt 2 gestartet wird, nachdem Schritt 1 und/oder Schritt 1a beendet sind, und wobei
Schritt 2 und/oder Schritt 1a und Schritt 2 solange durchgeführt werden, bis das Aerosol vollständig aus dem Reaktionsraum transportiert ist.

9. Vorrichtung zur Durchführung des Verfahrens nach einem der vorstehenden Ansprüche, mit:
wenigstens einer mikrostrukturierten Düse (1), deren Öffnung in einen Reaktionsraum (2) gerichtet ist, der größer als die Düse (1) ist; und
an dem Reaktionsraum (2) sind geeignete Mittel zum Transport (4) des Aerosols (AW) aus dem Reaktionsraum zu dessen Wirkort angeordnet; so daß die nachstehende Düsenstruktur bereitgestellt ist:
eine erste kleinere Düse (1) ist derart an einer zweiten größeren Düse (2) angeordnet, daß in dem Innenraum der größeren Düse (2) mittels der ersten Düse (1) eine Aerosolbildung in dem Innenraum (2) der größeren Düse erfolgt, und das Aerosol (AW) aus der zweiten Düse (2) zu seinem Wirkort ausbringbar ist.

10. Vorrichtung nach Anspruch 9, mit:
an dem Reaktionsraum (2) sind außerdem geeignete Mittel (3) angeordnet, um die weitere Diffusion des Aerosols (AW) nach dem Düsenaustritt mittels Bereitstellung von mechanischer und/oder elektromagnetischer Energie zu erzeugen.

11. Verfahren nach Anspruch 10, mit:
das Mittel zur Druckerzeugung ist ein der Düsenöffnung geeignet vorgeschalteter piezoelektrische Aktor (15);
zum Zuführen der Wirkstoffe sind den Düsen vorgeschaltete geeignet ausgebildete flexible Behälter (16) angeordnet, die ein Nachdringen von Luft bei absinkendem Flüssigkeitstand ausschließen;
als Mittel (3) zur weiteren Diffusion des Aerosols (AW) sind an dem Reaktionsraum (2) ein geeigneter Druckluft- und/oder Ultraschall und/oder Elektronenstrom und/oder Mikrowellengenerator angeordnet;
als Mittel zum Transport (4) ist an dem Reaktionsraum ist außerdem ein Ventilator und/oder Druckluftgenerator angeordnet, der einen Luftstrom zum Transport des Aerosols aus dem Reaktionsraum bereitstellt; und
die Vorrichtung weist außerdem eine geeignete elektronische Schaltung (6) zur Steuerung der Druckerzeugungsmittel (15), Mittel zur weiteren Diffusion (3) und Mittel zum Transport (4) auf.

12. Vorrichtung nach einem der Ansprüche 9 bis 11, mit:
die Düse (1) hat wenigstens zwei Düsenöffnungen (11, 12), die so ausgerichtet sind, daß aus den Düsenöffnungen austretende Strahlen des flüssigen Mediums in der Nähe der Düsenöffnungen in einem Kollisionspunkt (K) aufeinander treffen.

13. Vorrichtung nach einem der Ansprüche 9 bis 11, mit:
die Düse (10) hat wenigstens zwei Düsenöffnungen (11, 12), die so angeordnet sind, daß ein aus einer Düsenöffnung (11, 12) austretender Strahl des flüssigen Mediums und ein aus einer zweiten Düsenöffnung (11, 12) austretender Druckluftstrahl in der Nähe der Düsenöffnungen in einem Kollsionspunkt (K) aufeinander treffen.

14. Vorrichtung nach einem der Ansprüche 12 und 13, mit:
die Düse (10) hat wenigstens drei Düsenöffnungen (11, 12, 13), die so ausgebildet sind, daß aus zwei Düsenöffnungen (11, 12) austretende Strahlen des flüssigen Mediums und ein aus einer anderen Düsenöffnungen (13) austretender Druckluftstrahl in einem Kollisionspunkt (K) aufeinander treffen.

15. Vorrichtung nach einem der Ansprüche 11 bis 14, mit:
der Druckluft- und/oder Ultraschall- und/oder Elektronenstrom- und/oder Mikrowellengenerator ist in Übereinstimmung mit der jeweiligen Eigenfrequenz des flüssigen Mediums ausgebildet, so daß innerhalb kurzer Zeit eine weitere Diffusion des Aerosols (AW) erfolgt.

16. Vorrichtung nach einem der Ansprüche 11 bis 15, mit:
der Reaktionsraum (2) ist in Abhängigkeit von der Struktur und Anordnung der Düse (10) und dem mechanischen, elektromagnetischen und fluidischen Eigenschaften der Flüssigkeit, hinreichend dimensioniert ausgebildet, so daß das Aerosol (AW) an seiner inneren Wand nicht niederschlägt; und
der Reaktionsraum ist (2) auf die Frequenz des Ultraschall- und/oder Elektronenstrom- und/oder Mikrowellengenerator geeignet abgestimmt ausgebildet, so daß eine stehende Welle innerhalb des Reaktionsraums (2) ausbildbar ist.

17. Vorrichtung nach einem der Ansprüche 9 bis 16, mit:
der Reaktionsraum (2) besteht aus leitfähigem Material und seine innere Wand besteht aus duftinertem und/oder passiviertem Material.

18. Vorrichtung nach einem der Ansprüche 9 bis 17, mit einer Vielzahl von unabhängig ansteuerbaren Bauteilen (MEMS), die zumindest die Düse (1) und den Aktor (15) umfassen, und die derart ausgebildet sind, daß in das Innere der Bauteile (MEMS) keine Luft gelangt, und die linear, zirkular oder spiralförmig an dem Reaktionsraum angeordnet sind.

19. Vorrichtung nach einem der Ansprüche 9 bis 18, mit weiteren Düsen (1) zur Ausbringung von Druckluft, die an dem Reaktionsraum (2) angeordnet sind, so daß ein Luftstrom über der inneren Wand des Reaktionsraums (2) bereitstellbar ist, so daß eine Benetzung der inneren Wand weiter verhindert wird.

20. Vorrichtung nach einem der Ansprüche 9 bis 19, mit:
einer Vielzahl von 2 bis 1000 verschiedenen Duftstoffen und/oder Duftvernichtungsstoffen, die in voneinander getrennten Vorratsbehältern (16) untergebracht sind, wobei für jeden Duft und/oder Duftvemichter jeweils ein Bauteil (MEMS) zur Ausbringung des Duftstoffs oder Duftvernichters zur Verfügung steht.

21. Vorrichtung nach einem der Ansprüche 9 bis 20, außerdem mit einer elektronischen Steuerung (6), die derart ausgebildet ist, daß
die Ausbringungsmenge von Duftstoffen pro Bauteil (MEMS) pro Arbeitsintervall zwischen 1 Pikogramm und 100 Milligram beträgt,
die Zeitdauer des Arbeitsintervalls der Duftausbringung in einer sehr großen Bandbreite regelbar ist, wobei die Zeitfolge zwischen zwei Duftausbringungen sehr kurz oder Null sein kann, so daß auch Düfte gezielt mischbar sind;
die Steuerung über elektronische Impulse oder Programme erfolgen kann, wobei die Steuerung auch mit einer Rückkopplungsfunktion ausstattbar ist und über einen Sensor aktivierbar ist;
die Steuerung eine vorbestimmte Menge und/oder Zusammensetzung des Dufts bewirkt;
die Ausbringung synchron zu anderen Ereignissen erfolgen kann;
die Ausbringung eine Zeitvorwahl aufweisen kann;
die periodische Ausbringung periodisch einstellbar sein kann.

22. Vorrichtung nach einem der Ansprüche 9 bis 21, mit:
die Vorrichtung ist ein tragbares und/oder fest installierbares Gerät, das im Wohnbereich und/oder im Büro und/oder im Haushalt und/oder am Arbeitsplatz und/oder in öffentlichen Räumen und/oder in der Gastronomie und/oder für Promotionszwecke, z.B. im Handel zur Verbesserung der Raumluft durch Ausbringung von Dufstoffen und anderen Stoffen eingesetzt werden kann.

23. Vorrichtung nach einem der Ansprüche 9 bis 22, wobei:
die Vorrichtung ist ein tragbares und/oder fest installierbares Gerät, das Duftstoffe und andere Stoffe synchron zu anderen medialen Ereignissen ausbringt.

24. Vorrichtung nach einem der Ansprüche 9 bis 23, wobei:
die Vorrichtung ist mit anderen elektrischen oder elektronischen Geräten koppelbar ausgebildet, oder derart ausgebildet, daß
die Vorrichtung mit anderen elektrischen oder elektronischen Geräten kommunizieren kann; oder
über andere elektrische oder elektronische Geräte steuerbar ist; oder
die Vorrichtung bildet eine Einheit mit anderen elektrischen oder elektronischen Geräten, oder ist in andere elektrische oder elektronische Geräte integriert, wobei
die anderen Geräte bevorzugt Geräte sind, die in der Kommunikations- und Informationstechnik eingesetzt werden, wie insbesondere Fernseher, Radio, Telefon, Mobiltelefon, Computer; und/oder wobei
die anderen Geräte insbesondere Geräte sind, die in der Hygiene-, Umwelt-, Haus-, Fahrzeug- und Klimatechnik eingesetzt werden, wie insbesondere Desinfektionsgeräte, Klimageräte, Verdunstungs-Geräte, Zerstäubungsgeräte, Befeuchtungsgeräte.
